# EUROPÄISCHE PATENTSCHRIFT

(11) **EP 3 383 355 B1**
(45) Veröffentlichungstag und Bekanntmachung des Hinweises auf die Patenterteilung: **14.10.2020**
(21) Anmeldenummer: 16805409.6
(22) Anmeldetag: 01.12.2016
(51) Int. Cl.: A61K 8/34, A61K 8/35, A61Q 19/00, A61Q 19/08

(54) **EINSATZ VON ADDITIVEN ZUR EFFEKTIVEN KONSERVIERUNG / BOOSTENDE WIRKUNG BESTEHENDER KONSERVIERUNG KOSMETISCHER EMULSIONEN**
USE OF ADDITIVES FOR EFFECTIVE PRESERVATION/BOOST EFFECT OF EXISTING PRESERVATION OF COSMETIC EMULSIONS
UTILISATION D'ADDITIFS POUR CONSERVATION EFFICACE/EFFET AMÉLIORANT LA CONSERVATION EXISTANTE D'ÉMULSIONS COSMÉTIQUES

(30) Priorität: 04.12.2015 DE 102015224334
(43) Veröffentlichungstag der Anmeldung: 10.10.2018
(73) Patentinhaber: Henkel AG & Co. KGaA, 40589 Düsseldorf (DE)
(72) Erfinder: HARTMANN, Ira, 40589 Düsseldorf (DE); HEINEN, Soraya, 50858 Köln (DE); WALDMANN-LAUE, Marianne, 40789 Monheim (DE)
(86) Internationale Anmeldenummer: PCT/EP2016/079489
(87) Internationale Veröffentlichungsnummer: WO 2017/093428

(56) Entgegenhaltungen:
- EP-A1- 2 774 481
- CN-A- 104 546 522
- DE-A1- 19 649 287

## Beschreibung

Die vorliegende Erfindung betrifft neue Konservierungsmittelgemische und deren Einsatz auf dem Gebiet der Kosmetik.

Durch ihre Zusammensetzung können kosmetische Mittel als Nährmedium für Keime und Mikroorganismen dienen. Diese Keime können einerseits eine mikrobielle Kontamination des Verbrauchers herbeiführen, andererseits können sie Inhaltsstoffe der Kosmetika verändern und dabei Stoffe mit unerwünschten Wirkungen wie Sensibilisierung oder Hautreizung bilden. Um diese unerwünschten Folgen zu verhindern und um eine bestimmte Mindesthaltbarkeit der Kosmetika zu gewährleisten, müssen diese konserviert werden. Da Konservierungsmittel ihrerseits ein irritatives Potential besitzen, ist ihr Einsatz in Kosmetika streng reglementiert.

Die Hautmikroflora hat einen entscheidenden Einfluss auf unterschiedliche kosmetische Parameter. So spielen pathogene Keime wie Staphylococcus aureus eine entscheidende Rolle bei der Entstehung von Hautunreinheiten. Neueste Studien deuten auch darauf hin, dass eine unausgeglichene Hautmikroflora einen Einfluss auf die Hautalterung ausüben kann, da unerwünschte Keime zu einer gesteigerten Immunabwehr der Haut führen, die ihrerseits zu vermehrten Entzündungsreaktionen führt, in deren Verlauf Hautalterungsmarker stimuliert werden.

Es besteht daher nach wie vor der Bedarf nach Konservierungsmittelzusammensetzungen, die einerseits die Besiedlung des Produktes oder der Haut mit unerwünschten Keimen verhindern und andererseits die natürliche Hautflora nicht oder nicht wesentlich beeinträchtigen.

Die Mischung verschiedener antimikrobieller Substanzen zur Steigerung der antimikrobiellen Aktivität ist grundsätzlich bekannt. Beispielsweise offenbart die DE 10 2013 226874 A1 verschiedene Kombinationen von Ethylhexylglycerin (3-(2-Ethylhexyloxy)propan-1,2-diol mit anderen Konservierungsmitteln wie Phenoxyethanol und/oder Hexandiol, wobei die Beispielzusammensetzungen in diesem Dokument jeweils noch Ethyllauroylarginat enthalten. Weiterhin betrifft EP 2774481 A1 die Verwendung von Acetophenonderivaten, insbesondere 4-Hydroxyacetophenon, als antimikrobielles Mittel, welches bevorzugt in Kombinationen mit anderen antimikrobiellen Mitteln eingesetzt wird, wie beispielsweise Phenoxyethanol oder Ethylhexylglycerin. EP 2807925 A1 offenbart die Verwendung von Glycerylethern als Konservierungsmittel, bevorzugt in Kombination mit anderen Verbindungen, unter anderem mit 4-Hydroxyacetophenon. Von Hydroxyacetophenon ist auch bekannt, dass es die Wirkung anderer Konservierungsmittel wie Phenoxyethanol und von Formaldehyd-Donoren verstärken kann (S. Datenblatt SymSace® H, Symrise). DE 19649287 A1 offenbart 1,6-Hexandiol zur Konservierung wässriger kosmetischer Zusammensetzungen, gegebenenfalls in Kombination mit anderen Konservierungsmitteln.

Auch CN 104 546 522 A befasst sich mit Kombinationen von Konservierungsmitteln umfassend Hydroxyacetophenone.

Es besteht jedoch weiter der Bedarf, antimikrobielle Zusammensetzungen bereitzustellen, die bei geringer Einsatzmenge hocheffektiv sind. Der vorliegenden Erfindung lag die Aufgabe zugrunde, synergistische Wirkstoffkombinationen aufzufinden, die in geringen Konzentrationen hochwirksam sind und/oder aufgrund insgesamt verringerter Einsatzmengen die Herstellung reiz- und sensibilisierungsarmer Kosmetika ermöglichen.

Es wurde nun überraschend gefunden, dass diese Aufgabe durch Konservierungsmittelkombinationen aus 1,6-Hexandiol und Hydroxyacetophenon gelöst werden kann, wenn diese in definierten Konzentrationen in kosmetischen Mitteln vorliegen.

Durch die vorliegende Erfindung wird also bereitgestellt:
1. Eine kosmetische Zusammensetzung, welche als Konservierungsmittel enthält:
   (a) 0,1 bis 1,5 Gew.-% Hydroxyacetophenon, und
   (b) 3 bis 12 Gew.-% 1,6-Hexandiol,
   wobei sich die Gewichtsangaben jeweils auf das Gesamtgewicht der kosmetischen Zusammensetzung beziehen.
2. Die Verwendung von 1,6-Hexandiol in Kombination mit Hydroxyacetophenon als Konservierungsmittelsystem für kosmetische Zusammensetzungen, wobei die Endkonzentration von 1,6-Hexandiol in der kosmetischen Zusammensetzung 3 bis 12 Gew.-%, bevorzugt 5 bis 7 Gew.-%, weiter bevorzugt 6 Gew.-%, und die Endkonzentration von Hydroxyacetophenon 0,1 bis 1,5 Gew.-%, bevorzugt 0,2 bis 0,8 Gew.-%, beträgt und wobei das Konservierungsmittelsystem optional neben Hydroxyacetophenon und 1,6-Hexandiol weiterhin 2-Phenoxyethanol, Ethylhexylglycerin oder eine Kombination davon enthält.
3. Die Verwendung von 1,6-Hexandiol und Hydroxyacetophenon, wahlweise in Kombination mit Ethylhexylglycerin oder 2-Phenoxyethanol, zur Abtötung von oder zur Hemmung und/oder Verhinderung des Wachstums von Bakterien und Pilzen in kosmetischen Mitteln, wobei die Endkonzentration von 1,6-Hexandiol in der kosmetischen Zusammensetzung 3 bis 12 Gew.-%" bevorzugt 5 bis 7 Gew.-%, weiter bevorzugt 6 Gew.-%, und die Endkonzentration von Hydroxyacetophenon 0,1 bis 1,5 Gew.-%, bevorzugt 0,2 bis 0,8 Gew.-%, beträgt.

Erfindungsgemäße kosmetische Zusammensetzungen enthalten 0,1 bis 1,5 Gew.-%, bevorzugt 0,2 bis 1,0 Gew.-%, weiter bevorzugt 0,2 bis 0,8 Gew.-% Hydroxyacetophenon, bezogen auf das Gesamtgewicht der kosmetischen Zusammensetzung. In bevorzugten Ausführungsformen der Erfindung, insbesondere wenn das kosmetische Mittel als Konservierungsmittel außer Hydroxyacetophenon und dem 1,6-Hexandiol keine weiteren Konservierungsmittel enthält, beträgt die Konzentration an Hydroxyacetophenon bevorzugt 0,4 bis 1,0 Gew.-%, weiter bevorzugt 0,5 bis 0,8 Gew.-%. In weiteren bevorzugten Ausführungsformen, insbesondere wenn neben 1,6-Hexandiol und Hydroxyacetophenon weitere Konservierungsmittel enthalten sind, bevorzugt Ethylhexylglycerin und/oder Phenoxyethanol, beträgt der Gehalt an Hydroxyacetophenon bevorzugt 0,2 bis 0,8 Gew.-%, weiter bevorzugt 0,2 bis 0,5 Gew.-%, ebenfalls bevorzugt 0,2 bis 0,4 Gew.-%.

Unter Hydroxyphenon wird erfindungsgemäß eine oder mehrere Verbindungen verstanden, die aus 2-Hydroxyacetophenon, 3-Hydroxyacetophenon und 4-Hydroxyacetophenon ausgewählt ist. Geeignet sind insbesondere Gemische von zwei oder drei dieser Verbindungen. Besonders bevorzugt wird als Hydroxyphenon vorwiegend 4-Hydroxyphenon, z. B. in einer Menge von 80 Gew.-% oder mehr bevorzugt 90 Gew.-% oder mehr des gesamten Hydroxyphenons, weiter bevorzugt nur 4-Hydroxyphenon eingesetzt. 4-Hydroxyphenon ist im Handel beispielsweise unter der Bezeichnung SymSave® H (Symrise) erhältlich.

Die erfindungsgemäße kosmetische Zusammensetzung enthält als weiteren wesentlichen Bestandteil (b) 1,6-Hexandiol in einer Gesamtmenge von 3 bis 12 Gew.-%, bezogen auf das Gesamtgewicht der kosmetischen Zusammensetzung. Weiter bevorzugt ist das 1,6-Hexandiol in einer Gesamtmenge von 4 bis 10 Gew.-%, weiter bevorzugt 5 bis 7 Gew.-%, noch bevorzugter 5,5 bis 6,5 Gew.-% enthalten, am bevorzugtesten etwa 6 Gew.-%.

Die erfindungsgemäßen kosmetischen Zusammensetzungen können in bevorzugten Ausführungsformen weitere Konservierungsmittel enthalten, bevorzugt 2-Phenoxyethanol, Ethylhexylglycerin oder eine Kombination davon. Insbesondere 2-Phenoxyethanol und/oder Ethylhexylglycerin können die synergistische Wirkung, die für die Kombination von 1,6-Hexandiol und Hydroxyacetophenon beobachtet wurde, noch verstärken, sodass insgesamt häufig eine geringere Menge an Konservierungsmitteln eingesetzt werden braucht. Bevorzugt enthält die erfindungsgemäße kosmetische Zusammensetzung also neben dem 1,6-Hexandiol und dem Hydroxyacetophenon weiterhin 0,2 bis 1,0 Gew.-%, bevorzugt 0,4 bis 0,9 Gew.-%, ebenfalls bevorzugt 0,5 bis 0,8 Gew.-% 2-Phenoxyethanol (Phenoxyethanol) als Komponente (c), bezogen auf das Gesamtgewicht der kosmetischen Zusammensetzung.
Bevorzugt enthält die erfindungsgemäße kosmetische Zusammensetzung neben dem 1,6-Hexandiol und dem Hydroxyacetophenon als Komponente (d) 0,05 bis 2,0 Gew.-%, bevorzugt 0,1 bis 1 Gew.-%, weiter bevorzugt 0,2 bis 0,5 Gew.-% Ethylhexylglycerin (3-(2-Ethylhexyloxy)propan-1,2-diol), bezogen auf das Gesamtgewicht der kosmetischen Zusammensetzung.

In weiteren bevorzugten Ausführungsformen der Erfindung kann als Konservierungsmittel weiterhin neben dem 1,6-Hexandiol und dem Hydroxyacetophenon auch eine Kombination von 2-Phenoxyethanol und Ethylhexylglycerin enthalten sein. In diesem Fall beträgt die Gesamtmenge von 2-Phenoxyethanol und Ethylhexylglycerin bevorzugt 0,1 bis 3,0 Gew.-%, weiter bevorzugt 0,2 bis 1,5 Gew.-%, besonders bevorzugt 0,3 bis 0,9 Gew.-%, bezogen auf das Gesamtgewicht der kosmetischen Zusammensetzung.

Es ist erfindungsgemäß bevorzugt, wenn die kosmetischen Zusammensetzungen neben dem 1,6-Hydroxyacetophenon (a) und dem 1,6-Hexandiol (b), und wahlweise den Komponenten (c) (Phenoxyethanol) und/oder (d) (Ethylhexylglycerin), so wenig wie möglich weitere Konservierungsmittel enthalten, beispielsweise 0,05 Gew.-% oder weniger, bevorzugt 0,01 Gew.-% oder weniger, noch bevorzugter keine weiteren Konservierungsmittel. In Ausführungsformen der Erfindung ist die Gegenwart weiterer Konservierungsmittel aber nicht ausgeschlossen.

Weiterhin beträgt die Gesamtkonzentration an Konservierungsmitteln erfindungsgemäß bevorzugt weniger als 13 Gew.-%, bevorzugt 10 Gew.-% oder weniger, weiter bevorzugt weniger als 8 Gew.-%, weiter bevorzugt weniger als 7 Gew.-%, bezogen auf das Gesamtgewicht der kosmetischen Zusammensetzung.

Weiterhin beträgt die Gesamtmenge an Konservierungsmitteln, ausgenommen 1,6-Hexandiol, in den erfindungsgemäßen kosmetischen Mitteln bevorzugt 0,1 bis 3 Gew.-%, weiter bevorzugt 0,2 bis 2,0 Gew.-%, noch bevorzugter 0,3 bis 1 Gew.-%, ebenfalls bevorzugt 0,4 bis 0,9 Gew.-%.

Die erfindungsgemäßen kosmetischen Mittel können beispielsweise solche zur Hautpflege (Badepräparate, Hautwasch- und -reinigungsmittel, Hautpflegemittel, Augenkosmetika, Lippenpflegemittel, Nagelpflegemittel, Intimpflegemittel, Fußpflegemittel) sein, solche mit spezieller Wirkung (Lichtschutzmittel, Hautbräunungsmittel, Depigmentierungsmittel, Desodorantien, Antihidrotika, Haarentfernungsmittel, Rasiermittel, Duftmittel), solche zur Zahn- und Mundpflege (Zahn- und Mundpflegemittel, Gebisspflegemittel, Prothesenhaftmittel) und solche zur Haarpflege (Haarwaschmittel, Haarpflegemittel, Haarverfestigungsmittel, Haarverformungsmittel, Mittel zur Farbänderung). Erfindungsgemäß bevorzugte kosmetische Mittel sind ausgewählt aus der Gruppe der Hautcremes, Hautlotionen, Deodorantien, Antitranspirantien, Duschgele, Duschbäder, Zahnreinigungsmittel, Mundwässer, Haarshampoos, Haarkonditionierer, konditionierenden Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen.

Besonders bevorzugt sind die erfindungsgemäßen Mittel aus Hautpflegeprodukten ausgewählt. Das Hautpflegeprodukt kann insbesondere in Form einer Hautcreme, einer Hautlotion, einer Hautmilch, eines Gels, insbesondere Hydrogels, oder eines Serums vorliegen. Unter dem Begriff "Serum" wird erfindungsgemäß ein Hautpflegeprodukt verstanden, insbesondere ein wässriges Gel, bei dem die Wirkstoffe in vergleichsweise hoher Konzentration vorliegen. Bevorzugt handelt es sich bei den Hautpflegeprodukten um Produkte zur Anwendung im Gesicht, besonders bevorzugt um ein Mittel zur Verminderung von Alterserscheinungen der Haut.

Die erfindungsgemäßen kosmetischen Mittel enthalten die erfindungsgemäße Konservierungsmittel-Zusammensetzung und weitere Wirk- und/oder Hilfsstoffe in einem für die jeweilige Produktform geeigneten kosmetischen Träger. Üblicherweise werden die erfindungsgemäßen kosmetischen Mittel als Lösungen, Emulsionen oder Suspensionen formuliert, wobei ein wässriger oder ein wässrig-alkoholischer Träger oft bevorzugt wird. Erfindungsgemäß besonders bevorzugte kosmetische Mittel enthalten mindestens 30 Gew.-%, bevorzugt mindestens 40 Gew.-%, weiter bevorzugt mindestens 50 Gew.-%, ebenfalls bevorzugt mindestens 60 Gew.-% Wasser.

Erfindungsgemäß bevorzugte Mittel sind Hautbehandlungsmittel. Vorteilhafterweise liegen erfindungsgemäße Hautbehandlungsmittel in Form einer flüssigen, fließfähigen oder festen Öl-in-Wasser-Emulsion, Wasser-in-ÖI-Emulsion, Mehrfach-Emulsion, insbesondere einer Öl-in-Wasser-in-ÖI- oder Wasser-in-ÖI-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet). Mikroemulsionen aus, die sich bei weiterer Erwärmung in Wasser-in-ÖI-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

Bevorzugte optionale Inhaltsstoffe der erfindungsgemäßen kosmetischen Mittel können über ihre Funktion definiert werden. Natürlich können manche Inhaltsstoffe auch multifunktionell sein. Bevorzugte Inhaltsstoffe der erfindungsgemäßen kosmetischen Mittel können sein: Absorptionsmittel, antimikrobielle Stoffe, Bindemittel, Bleichmittel, Chelatbildner, Emollientien, Emulgatoren/ Dispergatoren, Emulsionsstabilisatoren, Enthaarungsmittel und Wirkstoffe mit Haarwuchs inhibierender Wirkung, Feuchtigkeitsspender, Pflegekomponenten wie Öle und Wachse, Filmbildner, Farbstoffe, Konservierungsstoffe, Korrosionsschutzmittel, hautkühlende Wirkstoffe, pH-Wert-Regler/ Puffersubstanzen, Tenside/waschaktive Substanzen, Treibgase, Trübungsmittel, UV-Absorber/ Lichtfiltersubstanzen, Vergällungsmittel, Viskositätsregler. Vorgenannte Inhaltsstoffe können gemäß Ausführungsformen als einziger Zusatz in erfindungsgemäß bevorzugten Mitteln enthalten sein. Bevorzugt sind aber Kombinationen der vorgenannten Inhaltsstoffe in den erfindungsgemäß bevorzugten Mitteln enthalten.

Wenn eine Pflegekomponente enthalten ist, sind erfindungsgemäß geeignete Öle ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Cetyl-2-ethylhexanoat, 2-Hexyldecylstearat (z. B. Eutanol® G 16 S), 2-Hexyldecyllaurat, Isodecylneopentanoat, Isononylisononanoat, 2-Ethylhexylpalmitat (z. B. Cegesoft® C 24) und 2-Ethylhexylstearat (z. B. Cetiol® 868). Ebenfalls geeignet sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat.

Weitere erfindungsgemäß geeignete Öle sind ausgewählt aus natürlichen und synthetischen Kohlenwasserstoffen, wie beispielsweise aus Mineralölen, Paraffinölen, C₁₈-C₃₀-Isoparaffinen, insbesondere Isoeicosan, Polyisobutene und Polydecene, die beispielsweise unter der Bezeichnung Emery® 3004, 3006, 3010 oder unter der Bezeichnung Ethylflo® von Albemarle oder Nexbase® 2004G von Nestle erhältlich sind, weiterhin ausgewählt aus C₈-C₁₆-Isoparaffinen, insbesondere aus Isodecan, Isododecan, Isotetradecan und Isohexadecan sowie Mischungen hiervon, sowie 1,3-Di-(2-ethylhexyl)-cyclohexan (erhältlich z. B. unter dem Handelsnamen Cetiol® S von BASF).

Weitere erfindungsgemäß geeignete Öle sind ausgewählt aus den Benzoesäureestern von linearen oder verzweigten C8-22-Alkanolen. Besonders bevorzugt sind Benzoesäure-C12-C15-alkylester, z. B. erhältlich als Handelsprodukt Finsolv® TN, Benzoesäureisostearylester, z. B. erhältlich als Handelsprodukt Finsolv® SB, Ethylhexylbenzoat, z. B. erhältlich als Handelsprodukt Finsolv® EB, und Benzoesäureoctyldocecylester, z. B. erhältlich als Handelsprodukt Finsolv® BOD.

Weitere erfindungsgemäß geeignete Öle sind ausgewählt aus Fettalkoholen mit 6 - 30 Kohlenstoffatomen, die ungesättigt oder verzweigt und gesättigt oder verzweigt und ungesättigt sind. Die verzweigten Alkohole werden häufig auch als Guerbet-Alkohole bezeichnet, da sie nach der Guerbet-Reaktion erhältlich sind. Bevorzugte Alkoholöle sind 2-Hexyldecanol (Eutanol® G 16), 2-Octyldodecanol (Eutanol® G), 2-Ethylhexylalkohol und Isostearylalkohol.

Weitere geeignete Öle sind ausgewählt aus Mischungen aus Guerbetalkoholen und Guerbetalkoholestern, z.B. dem Handelsprodukt Cetiol® PGL (2-Hexyldecanol und 2-Hexyldecyllaurat).
Weitere erfindungsgemäß bevorzugte kosmetische Öle sind ausgewählt aus den Triglyceriden (= Dreifachestern des Glycerins) von linearen oder verzweigten, gesättigten oder ungesättigten, gegebenenfalls hydroxylierten C8-30-Fettsäuren. Ebenfalls geeignet ist die Verwendung natürlicher Öle, z.B. Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannesbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls und dergleichen sein. Geeignet sind aber auch synthetische Triglyceridöle, insbesondere Capric/ Caprylic Triglycerides, z. B. die Handelsprodukte Myritol® 318, Myritol® 331 (BASF) oder Miglyol® 812 (Hüls) mit unverzweigten Fettsäureresten sowie Glyceryltriisostearin mit verzweigten Fettsäureresten.

Weitere erfindungsgemäß geeignete kosmetische Öle sind ausgewählt aus den Dicarbonsäureestern von linearen oder verzweigten C2-C10-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß geeignete kosmetische Öle sind ausgewählt aus den Anlagerungsprodukten von 1 bis 5 Propylenoxid-Einheiten an ein- oder mehrwertige C8-22-Alkanole, wie Octanol, Decanol, Decandiol, Laurylalkohol, Myristylalkohol und Stearylalkohol, z. B. PPG-2-Myristylether und PPG-3-Myristylether (Witconol® APM).

Weitere erfindungsgemäß geeignete kosmetische Öle sind ausgewählt aus den Anlagerungsprodukten von mindestens 6 Ethylenoxid- und/oder Propylenoxid-Einheiten an ein- oder mehrwertige C3-22-Alkanole wie Glycerin, Butanol, Butandiol, Myristylalkohol und Stearylalkohol, die gewünschtenfalls verestert sein können, z. B. PPG-14-Butylether (Ucon Fluid® AP), PPG-9-Butylether (Breox® B25), PPG-10-Butandiol (Macol® 57), PPG-15-Stearylether (Arlamol® E) und Glycereth-7-diiso-nonanoat.

Weitere erfindungsgemäß geeignete kosmetische Öle sind ausgewählt aus den C8-C22-Fettalko-holestern einwertiger oder mehrwertiger C2-C7-Hydroxycarbonsäuren, insbesondere die Ester der Glycolsäure, Milchsäure, Äpfelsäure, Weinsäure, Citronensäure und Salicylsäure. Solche Ester auf Basis von linearen C14/15-Alkanolen, z. B. C12-C15-Alkyllactat, und von in 2-Position verzweigten C12/13-Alkanolen sind unter dem Warenzeichen Cosmacol® von der Firma Nordmann, Rassmann GmbH & Co, Hamburg, zu beziehen, insbesondere die Handelsprodukte Cosmacol® ESI, Cosmacol® EMI und Cosmacol® ETI.

Weitere erfindungsgemäß geeignete kosmetische Öle sind ausgewählt aus den symmetrischen, unsymmetrischen oder cyclischen Estern der Kohlensäure mit C₃₋₂₂-Alkanolen, C₃₋₂₂-Alkandiolen oder C₃₋₂₂-Alkantriolen, z. B. Dicaprylylcarbonat (Cetiol® CC) oder die Ester gemäß der Lehre der DE 19756454 A1, insbesondere Glycerincarbonat.

Weitere kosmetische Öle, die erfindungsgemäß geeignet sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Weitere kosmetische Öle, die erfindungsgemäß geeignet sind, sind ausgewählt aus den Siliconölen, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Geeignet können flüchtige Siliconöle sein, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind. Ebenfalls geeignet sind flüchtige lineare Siliconöle, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/ oder L₄, bevorzugt solche Mischungen, wie sie z. B. in den Handelsprodukten DC 2-1184, Dow Corning® 200 (0,65 cSt) und Dow Corning® 200 (1,5 cSt) von Dow Corning enthalten sind. Geeignete nichtflüchtige Siliconöle sind ausgewählt aus höhermolekularen linearen Dimethylpolysiloxanen, im Handel erhältlich z. B. unter der Bezeichnung Dow Corning® 190, Dow Corning® 200 Fluid mit kinematischen Viskositäten (25°C) im Bereich von 5 - 100 cSt, bevorzugt 5 - 50 cSt oder auch 5 - 10 cSt, und Dimethylpolysiloxan mit einer kinematischen Viskosität (25°C) von etwa 350 cSt.

Als Pflegestoff kann das Mittel beispielsweise auch mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Geeignete Wachse sind bspw. ausgewählt aus Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Carbonsäuren, z. B. gehärteten Triglyceridfetten, Phospholipiden, beispielsweise Sojalecithin, Ei-Lecithin und Kephalinen.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Weiterhin können die erfindungsgemäß verwendeten kosmetischen Zusammensetzungen mindestens eine oberflächenaktive Substanz als Emulgator oder Dispergiermittel enthalten. Der Einsatz einer oberflächenaktiven Substanz ist besonders bevorzugt, wenn die erfindungsgemäß verwendeten kosmetischen Zusammensetzungen in Form von Emulsionen oder Dispersionen vorliegen.

### Geeignete Emulgatoren sind beispielsweise

- Anlagerungsprodukte von 4 bis 30 Mol Ethylenoxid und/oder 1 bis 5 Mol Propylenoxid an lineare C₈-C₂₂-Alkohole, an C₁₂-C₂₂-Carbonsäuren und an C₈-C₁₅-Alkylphenole,
- C₁₂-C₂₂-Carbonsäuremono- und -diester von Anlagerungsprodukten von 1 bis 30 Mol Ethylenoxid an C₃-C₆-Polyole, insbesondere an Glycerin,
- Ethylenoxid- und Polyglycerin-Anlagerungsprodukte an Methylglucosid-Carbonsäureester, Carbonsäurealkanolamide und Carbonsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Alkoholen, z. B. das im Handel erhältliche Produkt Montanov® 68,
- Anlagerungsprodukte von 5 bis 60 Mol Ethylenoxid an Rizinusöl und gehärtetes Rizinusöl,
- Partialester von Polyolen mit 3 bis 6 Kohlenstoffatomen mit gesättigten C₈-C₂₂-Carbonsäuren,
- Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide,
- Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls® PGPH) und Polyglyceryl-3-diisostearat (Handelsprodukt Lameform® TGI) sowie
- lineare und verzweigte C₈-C₃₀-Carbonsäuren und deren Na-, K-, Ammonium-, Ca-, Mg- und Zn - Salze.

Weiterhin können jedoch auch oberflächenaktive Substanzen in Form von nichtionischen Emulgatoren mit HLB-Werten von 8 und darunter in der erfindungsgemäß verwendeten kosmetischen Zusammensetzung enthalten sein. Derartige bevorzugte Emulgatoren sind insbesondere Verbindungen der allgemeinen Formel R¹ - O - R², in welcher R¹ eine primäre lineare Alkyl-, Alkenyl- oder Acylgruppe mit 20 bis 30 Kohlenstoffatomen, bevorzugt ein Behenyl-Rest, und R² Wasserstoff, eine Gruppe der Formel -(CₙH₂ₙO)ₓ-H mit x = 1 oder 2 und n = 2 bis 4 oder eine Polyhydroxyalkylgruppe mit 4 bis 6 Kohlenstoffatomen und 2 bis 5 Hydroxylgruppen, bevorzugt Wasserstoff, darstellt. Eine besonders bevorzugte Verbindung R¹ - O - R² ist Behenylalkohol. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Carbonsäuren mit Polyolen wie z. B. Pentaerythrit, Trimethylolpropan, Diglycerin, Sorbit, Glucose oder Methylglucose. Beispiele für solche Produkte sind beispielsweise Sorbitanmonobehenat oder Pentaerythrit-monoerucat. Die Anwesenheit von Emulgatoren der allgemeinen Formel R¹ - O - R² kann zu lamellaren Strukturen in der Emulsion führen, welche bei der erfindungsgemäßen Verwendung besonders günstige Effekte auf die Wiederherstellung der lamellaren Struktur der Haut haben können.

Weitere geeignete Zusatzstoffe in den erfindungsgemäß verwendeten kosmetischen Zusammensetzungen sind Verdickungsmittel, wie
- natürliche und synthetische Tone und Schichtsilikate, wie Bentonit, Hectorit, Montmorillonit oder Laponite®,
- anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel®305, Simulgel®600, Simulgel® NS, Simulgel® EPG und Simulgel® EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol®. Ein besonders bevorzugtes anionisches Copolymer enthält als Monomer 80 bis 98 % einer ungesättigten, gegebenenfalls substituierten C₃₋₆-Carbonsäure oder ihres Anhydrids sowie 2 bis 20 % eines gegebenenfalls substituierten Acrylsäureesters von gesättigten C₁₀₋₃₀-Carbonsäuren, wobei das Copolymer mit den vorgenannten Vernetzungsagentien vernetzt sein kann. Entsprechende Handelsprodukte sind Pemulen® und die Carbopol®-Typen 954, 980, 1342 und ETD 2020 (ex B.F. Goodrich).
- Geeignete nichtionische Polymere sind beispielsweise Polyvinylalkohole, welche teilverseift sein können, z. B. die Handelsprodukte Mowiol® sowie Vinylpyrrolidon/Vinylester-Copolymere und Polyvinylpyrrolidone, die z. B. unter dem Warenzeichen Luviskol® (BASF) vertrieben werden.

Weitere bevorzugte Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum®, Parfümöle, Pigmente sowie Farbstoffe zum Anfärben der kosmetischen Zusammensetzung, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft.

### Tabellarische Übersicht

Die Zusammensetzung von Konservierungsmittelkombinationen einiger bevorzugter kosmetischer Mittel kann den folgenden Tabellen entnommen werden (Angaben als Aktivstoffgehalt und in Gew.-% bezogen auf das Gesamtgewicht des kosmetischen Mittels).

| | Formel 1 | Formel 2 | Formel 3 | Formel 4 |
|---|---|---|---|---|
| (a) Hydroxyacetophenon | 0,1 bis 1,5 | 0,2 bis 1 | 0,4 bis 0,9 | 0,5 bis 0,8 |
| (b) 1,6-Hexandiol | 3 bis 12 | 4 bis 10 | 5 bis 7 | 5,5 bis 6,5 |
| Misc | add 100 | add 100 | add 100 | add 100 |

| | Formel 5 | Formel 6 | Formel 7 | Formel 8 |
|---|---|---|---|---|
| (a) Hydroxyacetophenon | 0,1 bis 1,5 | 0,2 bis 0,8 | 0,2 bis 0,5 | 0,2 bis 0,4 |
| (b) 1,6-Hexandiol | 3 bis 12 | 4 bis 10 | 5 bis 7 | 5,5 bis 6,5 |
| (c) 2-Phenoxyethanol | 0,2 bis 1 | 0,3 bis 0,9 | 0,4 bis 0,8 | 0,5 bis 0,7 |
| Misc | add 100 | add 100 | add 100 | add 100 |

| | Formel 9 | Formel 10 | Formel 11 | Formel 12 |
|---|---|---|---|---|
| (a) Hydroxyacetophenon | 0,1 bis 1,5 | 0,2 bis 0,8 | 0,2 bis 0,5 | 0,2 bis 0,4 |
| (b) 1,6-Hexandiol | 3 bis 12 | 4 bis 10 | 5 bis 7 | 5,5 bis 6,5 |
| (d) Ethylhexylglycerin | 0,05 bis 2 | 0,1 bis 1 | 0,2 bis 0,5 | 0,2 bis 0,5 |
| Misc | add 100 | add 100 | add 100 | add 100 |

| | Formel 13 | Formel 14 | Formel 15 | Formel 16 |
|---|---|---|---|---|
| (a) Hydroxyacetophenon | 0,1 bis 1,5 | 0,2 bis 0,8 | 0,2 bis 0,5 | 0,2 bis 0,4 |
| (b) 1,6-Hexandiol | 3 bis 12 | 4 bis 10 | 5 bis 7 | 5,5 bis 6,5 |
| (c) 2-Phenoxyethanol | 0,2 bis 1 | 0,3 bis 0,9 | 0,4 bis 0,8 | 0,5 bis 0,7 |
| (d) Ethylhexylglycerin | 0,05 bis 2 | 0,1 bis 1 | 0,2 bis 0,5 | 0,2 bis 0,5 |
| Misc | add 100 | add 100 | add 100 | add 100 |

Unter "Misc" sind erfindungsgemäß kosmetische Träger, insbesondere Wasser, und weitere übliche Bestandteile kosmetischer Mittel zu verstehen, bspw. Parfüms/Duftstoffe, Pflegekomponenten, Emulgatoren, Verdickungsmittel, pH-Regulatoren. Bevorzugt fallen unter "Misc" 0,05 Gew.-% oder weniger, 0,01 Gew.-% oder weniger und am vorteilhaftesten keine weiteren Konservierungsmittel.

### Beispiele

### Beispiele 1 bis 5 und Vergleichsbeispiele 1 bis 3:

Es wurden folgende Gesichtscremes (Faltencremes) hergestellt:

| **Komponente/Rohstoff** | **V1** | **V2** | **Bsp.1** | **Bsp.2** | **V3** | **Bsp.3** | **Bsp.4** | **Bsp.5** |
|---|---|---|---|---|---|---|---|---|
| Lipoid S 75-3 | 0,25 | 0,25 | 0,25 | 0,25 | 0,25 | 0,25 | 0,25 | 0,25 |
| Glycerin 99,5% | 5,0 | 5,0 | 5,0 | 5,0 | 5,0 | 5,0 | 5,0 | 5,0 |
| Cocosglyceride C12-18 | 4,0 | 4,0 | 4,0 | 4,0 | 4,0 | 4,0 | 4,0 | 4,0 |
| 2-Ethylhexyl palmitate | 2,0 | 2,0 | 2,0 | 2,0 | 2,0 | 2,0 | 2,0 | 2,0 |
| Vitamin E Acetate | 0,5 | 0,5 | 0,5 | 0,5 | 0,5 | 0,5 | 0,5 | 0,5 |
| Dimethicone 5 cSt | 2,0 | 2,0 | 2,0 | 2,0 | 2,0 | 2,0 | 2,0 | 2,0 |
| Aristoflex® AVC | 0,2 | 0,2 | 0,2 | 0,2 | 0,2 | 0,2 | 0,2 | 0,2 |
| D.S.H. C N® 6 | 0,9 | 0,9 | 0,9 | 0,9 | 0,9 | 0,9 | 0,9 | 0,9 |
| Fucocert® | 5,0 | 5,0 | 5,0 | 5,0 | 5,0 | 5,0 | 5,0 | 5,0 |
| Ridulisse® C GR | 2,0 | 2,0 | 2,0 | 2,0 | 2,0 | 2,0 | 2,0 | 2,0 |
| Lys Lastine® V | 0,5 | 0,5 | 0,5 | 0,5 | 0,5 | 0,5 | 0,5 | 0,5 |
| Hydroxyprolisilane SPE | 1,0 | 1,0 | 1,0 | 1,0 | 1,0 | 1,0 | 1,0 | 1,0 |
| Vegetal Micropatch (A00244) | 2,0 | 2,0 | 2,0 | 2,0 | 2,0 | 2,0 | 2,0 | 2,0 |
| Parfum Nice Orris 12/07 | 0,05 | 0,05 | 0,05 | 0,05 | 0,05 | 0,05 | 0,05 | 0,05 |
| Taurine | 1,0 | 1,0 | 1,0 | 1,0 | 1,0 | 1,0 | 1,0 | 1,0 |
| Herbasec Eyebright | 1,5 | 1,5 | 1,5 | 1,5 | 1,5 | 1,5 | 1,5 | 1,5 |
| Simulgel® EPG | 2,0 | 2,0 | 2,0 | 2,0 | 2,0 | 2,0 | 2,0 | 2,0 |
| Keltrol® CG-SFT | 0,3 | 0,3 | 0,3 | 0,3 | 0,3 | 0,3 | 0,3 | 0,3 |
| | | | | | | | | |
| 1,6-Hexandiol | 6,0 | 6,0 | 6,0 | 6,0 | - | 6,0 | 6,0 | 6,0 |
| Phenoxyethanol, pure | 0,5 | 0,9 | 0,5 | 0,5 | 0,5 | - | - | - |
| SymSave® H | - | - | 0,2 | 0,4 | - | 0,4 | 0,4 | 0,8 |
| Sensiva® SC 50 | - | - | - | - | - | - | 0,2 | - |
| Isopentyldiol | - | - | - | - | 6,0 | - | - | - |
| Wasser, vollentsalzt | ad 100 | ad 100 | ad 100 | ad 100 | ad 100 | ad 100 | ad 100 | ad 100 |
| Gesamt: | 100 | 100 | 100 | 100 | 100 | 100 | 100 | 100 |

Die Mengenangaben in den Tabellen sind in Gew.-% des jeweiligen Rohstoffs, bezogen auf die gesamte Zusammensetzung, angegeben.

Erläuterung der Inhaltsstoffe (INCI oder andere Bezeichnung, ggf. Hersteller):
- Lipoid S 75-3:: Hydrogenated Lecithin (Lipoid Kosmetik)
- Aristoflex® AVC:: Ammonium Acryloyldimethyltaurate/VP Copolymer (Clariant)
- D.S.H. C N® 6.: Water, Dimethylsilanol Hyaluronate (Exsymol)
- Fucocert®:: Aqua, Biosaccharide Gum-1
- Ridulisse® C GR:: Water, HYDROLYZED SOY PROTEIN (3 - 5 Gew.-% Aktivsubstanz)
- Lys Lastine® V:: Dill seed extract (BASF)
- Hydroxyprolisilane SPE:: Methylsilanol hydroxyproline aspartate (Exsymol)
- Vegetal Micropatch:: Aqua (Water), Butylene Glycol,Pentylene Glycol, Algin, Acacia Senegal Gum, Serine
- Herbasec Eyebright:: Maltodextrin, Euphrasia Officinalis Extract
- Simulgel® EPG:: Sodium Acrylate/Sodium Acryloyldimethyl Taurate Copolymer (and) Polyisobutene (and) Caprylyl/Capryl Glucoside (Seppic)
- Keltrol® CG-SFT:: Xanthan Gum (CP Kelco)
- SymSave® H.: 4-Hydroxyacetophenon (Symrise), mind. 99 %
- Sensiva® SC 50:: Ethylhexyglycerine (Schülke), mind. 99 %

### Konservierungsmittelbelastungstests

Zur Prüfung der ausreichenden Konservierung wurden die Zusammensetzungen gemäß den Beispielen 1 bis 5 und den Vergleichsbeispielen V1 bis V3 einem Konservierungsmittelbelastungstest in Anlehnung an Ph. Eur. (Europäisches Arzneibuch), 6. Ausgabe, 5.3.1 unterzogen.

Hierfür wurden 30 g der zu testenden Zusammensetzung jeweils mit 10⁵ koloniebildenden Einheiten (KBE) pro 1 g Zusammensetzung der folgenden Testmikroorganismen geimpft: Pseudomonas aeruginosa (Bakterium), Staphylococcus aureus (Bakterium), Candida albicans (Pilz), Aspergillus brasiliensis (Pilz). Nach Zugabe des jeweiligen Mikroorganismus wurde die Probe durch Rühren mittels eines Glasstabes homogenisiert und anschließend bei 20 bis 25 °C im Dunkeln gelagert. Nach 7, 14, 21 oder 28-tägiger Lagerung der geimpften Zusammensetzungen wurde jeweils 1 g der jeweiligen Probe entnommen und die darin enthaltenen KBE bestimmt.

Ziel der Konservierung ist eine Reduktion der KBE auf einen Wert unterhalb der Nachweisgrenze. Die in den Tabellen angegebenen Werte (7, 14, 28) geben die Anzahl der Tage an, nach denen die KBE unter die Nachweisgrenze gefallen war (d.h. 7 = nach 7 Tagen lag die Anzahl der KBE unterhalb der Nachweisgrenze).
Je schneller alle Keime abgetötet wurden, desto besser wirkte das Konservierungsmittel (d.h. das beste Ergebnis war 7 (Bakterien), 7 (Pilze)).
Die Ergebnisse sind in der folgenden Tabelle dargestellt.

| **Rezeptur** | **Konservieru ng** (zusätzlich zu 6 Gew.-% 1,6-Hexandiol) | **Summe Konservierung** (zusätzlich zu 6 Gew.-% 1,6-Hexandiol) | Bakterien | Pilze |
|---|---|---|---|---|
| V1 | 0,5 % Phenoxyethanol | 0,5 % | 28 | 21 |
| V2 | 0,9 % Phenoxyethanol | 0,9 % | 14 | 14 |
| Bsp. 1 | 0,2 % Hydroxyacetophenon* + 0,5 % Phenoxyethanol | 0,7 % | 14 | 7 |
| Bsp. 2 | 0,4 % Hydroxyacetophenon* + 0,5 % Phenoxyethanol | 0,9 % | 7 | 7 |
| V3 | 0,5 % Phenoxyethanol Isopentyldiol anstelle von 1,6-Hexandiol | 0,5 % | 28 | 28 |
| Bsp. 3 | 0,4 % Hydroxyacetophenon* | 0,4 % | 14 | 7 |
| Bsp. 4 | 0,4 % Hydroxyacetophenon* + 0,2 % Ethylhexylglycerin | 0,6 % | 7 | 7 |
| Bsp. 5 | 0,8 % Hydroxyacetophenon* | 0,8 % | 7 | 7 |

Prozentangaben jeweils in Gew.-%, bezogen auf das Gesamtgewicht der jeweiligen Rezeptur * eingesetzt als Symsave® H (4-Hydroxyacetophenon)

Im Fall der erfindungsgemäßen Konservierungsmittelkombinationen konnte jeweils eine deutliche Verbesserung hinsichtlich der antibakteriellen und fungiziden Wirkung beobachtet werden, teils bei verringerter Konservierungsmittelgesamtmenge (zum Beispiel Bsp. 4).

### Beispiele 6 und 7 und Vergleichsbeispiele 4 und 5

Es wurden folgende Tagescremes (Faltencremes) hergestellt:

| **Komponente/Rohstoff** | **V4** | **Bsp.6** | **V5** | **Bsp.7** |
|---|---|---|---|---|
| Isopropyl stearate | 4,0 | 4,0 | 4,0 | 4,0 |
| Cetiol® B | 2,0 | 2,0 | 2,0 | 2,0 |
| Vitamin E Acetate | 0,5 | 0,5 | 0,5 | 0,5 |
| Cutina® MD | 1,0 | 1,0 | 1,0 | 1,0 |
| Cetearylalkohol | 0,5 | 0,5 | 0,5 | 0,5 |
| Cocosglyceride C12-18 | 2,0 | 2,0 | 2,0 | 2,0 |
| Lanette® 22 | 2,0 | 2,0 | 2,0 | 2,0 |
| Lipoid S 75-3 | 1,0 | 1,0 | 1,0 | 1,0 |
| DC EL-8040 ID Silicone Organic Blend | 0,9 | 0,9 | 0,9 | 0,9 |
| Glycerin 99,5 % | 5,0 | 5,0 | 5,0 | 5,0 |
| 1,2-Propandiol | 5,0 | 5,0 | 5,0 | 5,0 |
| Tego® Carbomer 140 | 0,4 | 0,4 | 0,4 | 0,4 |
| Talc | 1,0 | 1,0 | 1,0 | 1,0 |
| Titanium Dioxide | 0,5 | 0,5 | 0,5 | 0,5 |
| FD&C Red No. 40 | 0,0001 | 0,0001 | 0,0001 | 0,0001 |
| Photosomes V | 0,2 | - | - | - |
| Photosomes | - | 0,2 | 0,2 | 0,2 |
| Ederline LS | 2,0 | 2,0 | 2,0 | 2,0 |
| D.S.H C N® 6 | 5,0 | 5,0 | 5,0 | 5,0 |
| Lipochroman® | 0,01 | 0,01 | 0,01 | 0,01 |
| Parfum Diamond Novel 49316332 | 0,3 | 0,3 | 0,3 | 0,3 |
| Matrixyl 3000 PEG free | 3,0 | 3,0 | 3,0 | 3,0 |
| RonaCare® AP | 0,1 | 0,1 | 0,1 | 0,1 |
| SUNPMMA-S | 1,0 | 1,0 | 1,0 | 1,0 |
| Simulgel® EPG | 1,5 | 1,5 | 1,5 | 1,5 |
| Sodium hydroxide pearls | 0,019 | 0,019 | 0,019 | 0,019 |
| | | | | |
| 1,6-Hexandiol | 6,0 | 6,0 | - | 6,0 |
| Phenoxyethanol, pure | 0,5 | 0,5 | 0,5 | 0,5 |
| SymSave® H | - | 0,2 | - | 0,4 |
| Sensiva® SC 50 | 0,2 | - | 0,2 | - |
| Isopentyldiol | - | - | 3,0 | - |
| | | | | |
| Wasser, vollentsalzt | ad 100 | ad 100 | ad 100 | ad 100 |
| **Gesamt:** | 100 | 100 | 100 | 100 |

Die Mengenangaben in den Tabellen sind in Gew.-% des jeweiligen Rohstoffs, bezogen auf die gesamte Zusammensetzung, angegeben.

Erläuterung der Inhaltsstoffe (INCI oder andere Bezeichnung, ggf. Hersteller):
- Cetiol® B:: Dibutyl Adipate (BASF)
- Cutina® MD:: Glyceryl Stearate (Mischung aus Glycerylmono- und distearat) (BASF)
- Lanette® 22:: Behenyl Alcohol (BASF)
- Lipoid S 75-3:: Hydrogenated Lecithin (Lipoid Kosmetik)
- DC EL-8040 ID Silicone: Silicone elastomer, Isododecane, Dimethicone Crosspolymer
- Organic Blend: Isododecane (and) Dimethicone Crosspolymer (Dow Corning)
- Tego® Carbomer 140:: Carbomer (Evonik)
- FD&C Red No. 40: Allurarot AC
- Photosomes V:: Aqua, Plankton extract, lecithin (ProTec)
- Photosomes:: Aqua, Plankton extract, lecithin (ProTec)
- Ederline LS:: 2-Hexyldecan-1-ol, 1,3-Butandiol, Pyrus Malus (Apple) Seed Extract
- D.S.H C N® 6:: Water, Dimethylsilanol Hyaluronate (Exsymol)
- Lipochroman®:: (Lipotec)
- Matrixyl 3000 PEG free:: Glycerin, Aqua (Water), Butylene Glycol, Carbomer, Coco Glucoside, Palmitoyl Tripeptide-1, Palmitoyl Tetrapeptide-7
- RonaCare® AP: Bis-Ethylhexyl Hydroxydimethoxy Benzylmalonate
- SUNPMMA-S:: Polymethyl Methacrylate (Sunjin Chemical)
- Simulgel® EPG:: Sodium Acrylate/Sodium Acryloyldimethyl Taurate Copolymer (and) Polyisobutene (and) Caprylyl/Capryl Glucoside (Seppic)
- SymSave® H.: Hydroxyacetophenon (Symrise)
- Sensiva® SC 50:: Ethylhexyglycerine (Schülke)

Es wurden wiederum Konservierungsmittelbelastungstests wie oben beschrieben durchgeführt. Die Ergebnisse finden sich in der nachfolgenden Tabelle:

| **Rezeptur** | **Konservieru ng** (zusätzlich zu 6 Gew.-% 1,6-Hexandiol) | **Summe Konservierung** (zusätzlich zu 6 Gew.-% 1,6-Hexandiol) | Bakterien | Pilze |
|---|---|---|---|---|
| V4 | 0,5 % Phenoxyethanol + 0,2 % Ethylhexylglycerin | 0,7 % | 7 | 28 |
| Bsp. 6 | 0,5 % Phenoxyethanol + 0,2 % Hydroxyacetophenon* | 0,7 % | 7 | 14 |
| V5 | 0,5 % Phenoxyethanol + 0,2 % Ethylhexylglycerin Isopentyldiol anstelle von 1,6-Hexandiol | 0,7 % | 7 | 28 |
| Bsp. 7 | 0,5 % Phenoxyethanol + 0,4 % Hydroxyacetophenon* | 0,9 % | 7 | 7 |

| | | | | |
|---|---|---|---|---|
| Prozentangaben jeweils in Gew.-%, bezogen auf das Gesamtgewicht der jeweiligen Rezeptur * eingesetzt als Symsave® H (4-Hydroxyacetophenon) | | | | |

Wiederum wurde im Fall der erfindungsgemäßen Konservierungsmittelkombinationen eine starke Verbesserung hinsichtlich der antibakteriellen und fungiziden Wirkung beobachtet.

## Patentansprüche

1. Kosmetische Zusammensetzung, welche als Konservierungsmittel enthält:
(a) 0,1 bis 1,5 Gew.-% Hydroxyacetophenon, bevorzugt 4-Hydroxyacetophenon, und
(b) 3 bis 12 Gew.-% 1,6-Hexandiol,
wobei sich die Gewichtsangaben jeweils auf das Gesamtgewicht der kosmetischen Zusammensetzung beziehen.

2. Kosmetische Zusammensetzung nach Anspruch 1, welche als Konservierungsmittel weiterhin (c) 0,2 bis 1,0 Gew.-%, bevorzugt 0,4 bis 0,9 Gew.-% 2-Phenoxyethanol enthält, bezogen auf das Gesamtgewicht der kosmetischen Zusammensetzung.

3. Kosmetische Zusammensetzung nach Anspruch 1 oder 2, welche als Konservierungsmittel weiterhin
(d) 0,05 bis 2,0 Gew.-%, bevorzugt 0,2 bis 0,5 Gew.-% Ethylhexylglycerin enthält, bezogen auf das Gesamtgewicht der kosmetischen Zusammensetzung.

4. Kosmetische Zusammensetzung nach einem der Ansprüche 1 bis 3, wobei der Gehalt an Hydroxyacetophenon 0,2 bis 1,0 Gew.-%, bevorzugt 0,2 bis 0,4 Gew.-% beträgt, bezogen auf das Gesamtgewicht der kosmetischen Zusammensetzung.

5. Kosmetische Zusammensetzung nach Anspruch 1, wobei als Konservierungsmittel nur 1,6-Hexandiol und Hydroxyacetophenon enthalten sind, und wobei der Gehalt an Hydroxyacetophenon bevorzugt 0,4 bis 0,8 Gew.-% beträgt, bezogen auf das Gesamtgewicht der kosmetischen Zusammensetzung.

6. Kosmetische Zusammensetzung nach einem der vorhergehenden Ansprüche, wobei der Gehalt an 1,6-Hexandiol (b) 5 bis 7 Gew.-% beträgt, bevorzugt 5,5 bis 6,5 Gew.-%, weiter bevorzugt 6 Gew.-%, bezogen auf das Gesamtgewicht der kosmetischen Zusammensetzung.

7. Kosmetische Zusammensetzung nach einem der vorhergehenden Ansprüche, wobei es sich bei der kosmetischen Zusammensetzung um ein kosmetisches Mittel zur Verminderung der Alterserscheinungen der Haut handelt.

8. Kosmetische Zusammensetzung nach einem der vorhergehenden Ansprüche, wobei die kosmetische Zusammensetzung 0,01 Gew.-% oder weniger, bevorzugt keine weiteren Konservierungsmittel enthält.

9. Verwendung von 1,6-Hexandiol in Kombination mit Hydroxyacetophenon als Konservierungsmittelsystem für kosmetische Zusammensetzungen, wobei die Endkonzentration von 1,6-Hexandiol in der kosmetischen Zusammensetzung 3 bis 12 Gew.-%, bevorzugt 5 bis 7 Gew.-%, weiter bevorzugt 6 Gew.-%, und die Endkonzentration von Hydroxyacetophenon 0,1 bis 1,5 Gew.-%, bevorzugt 0,2 bis 0,8 Gew.-%, beträgt, und wobei das Konservierungsmittelsystem optional neben Hydroxyacetophenon und 1,6-Hexandiol weiterhin 2-Phenoxyethanol, Ethylhexylglycerin oder eine Kombination davon enthält.

10. Verwendung von 1,6-Hexandiol und Hydroxyacetophenon, wahlweise in Kombination mit Ethylhexylglycerin oder 2-Phenoxyethanol, zur Abtötung von oder zur Hemmung und/oder Verhinderung des Wachstums von Bakterien und Pilzen in kosmetischen Mitteln, wobei die Endkonzentration von 1,6-Hexandiol in der kosmetischen Zusammensetzung 3 bis 12 Gew.-%" bevorzugt 5 bis 7 Gew.-%, weiter bevorzugt 6 Gew.-%, und die Endkonzentration von Hydroxyacetophenon 0,1 bis 1,5 Gew.-%, bevorzugt 0,2 bis 0,8 Gew.-%, beträgt.

## Claims

1. A cosmetic composition containing, as a preservative:
(a) 0.1 to 1.5 wt.% hydroxyacetophenone, preferably 4-hydroxyacetophenone; and
(b) 3 to 12 wt.% 1,6-hexanediol,
wherein the weight specifications relate in each case to the total weight of the cosmetic composition.

2. The cosmetic composition according to claim 1, which further contains, as a preservative
(c) 0.2 to 1.0 wt.%, preferably 0.4 to 0.9 wt.%, 2-phenoxyethanol, based on the total weight of the cosmetic composition.

3. The cosmetic composition according to claim 1 or 2, which further contains, as a preservative
(d) 0.05 to 2.0 wt.%, preferably 0.2 to 0.5 wt.%, ethylhexylglycerin, based on the total weight of the cosmetic composition.

4. The cosmetic composition according to one of claims 1 to 3, wherein the content of hydroxyacetophenone is 0.2 to 1.0 wt.%, preferably 0.2 to 0.4 wt.%, based on the total weight of the cosmetic composition.

5. The cosmetic composition according to claim 1, wherein only 1,6-hexanediol and hydroxyacetophenone are contained as preservatives, and wherein the content of hydroxyacetophenone is preferably 0.4 to 0.8 wt.%, based on the total weight of the cosmetic composition.

6. The cosmetic composition according to one of the preceding claims, wherein the content of 1,6-hexanediol (b) is 5 to 7 wt.%, preferably 5.5 to 6.5 wt.%, more preferably 6 wt.%, based on the total weight of the cosmetic composition.

7. The cosmetic composition according to one of the preceding claims, wherein the cosmetic composition is a cosmetic agent for reducing the signs of aging of the skin.

8. The cosmetic composition according to one of the preceding claims, wherein the cosmetic composition contains 0.01 wt.% or less of further preservatives, preferably no further preservatives.

9. The use of 1,6-hexanediol in combination with hydroxyacetophenone as a preservative system for cosmetic compositions, wherein the final concentration of 1,6-hexanediol in the cosmetic composition is 3 to 12 wt.%, preferably 5 to 7 wt.%, more preferably 6 wt.%, and the final concentration of hydroxyacetophenone is 0.1 to 1.5 wt.%, preferably 0.2 to 0.8 wt.%, and wherein the preservative system optionally further contains, in addition to hydroxyacetophenone and 1,6-hexanediol, 2-phenoxyethanol, ethylhexylglycerin or a combination thereof.

10. The use of 1,6-hexanediol and hydroxyacetophenone, optionally in combination with ethylhexylglycerin or 2-phenoxyethanol, to kill or inhibit and/or prevent the growth of bacteria and fungi in cosmetic agents, wherein the final concentration of 1,6-hexanediol in the cosmetic composition is 3 to 12 wt.%, preferably 5 to 7 wt.%, more preferably 6 wt.%, and the final concentration of hydroxyacetophenone is 0.1 to 1.5 wt.%, preferably 0.2 to 0.8 wt.%.

## Revendications

1. Composition cosmétique, qui contient comme agents conservateurs :
a) 0,1 à 1,5 % en poids d'hydroxyacétophénone, de préférence de 4-hydroxyacétophénone, et
b) 3 à 12 % en poids de 1,6-hexanediol,
les indications de poids se rapportant dans chaque cas au poids total de la composition cosmétique.

2. Composition cosmétique selon la revendication 1, qui contient en outre comme agent conservateur
(c) 0,2 à 1,0 % en poids, de préférence 0,4 à 0,9 % en poids de 2-phénoxyéthanol, par rapport au poids total de la composition cosmétique.

3. Composition cosmétique selon la revendication 1 ou 2, qui contient en outre comme agent conservateur
(d) 0,05 à 2,0 % en poids, de préférence 0,2 à 0,5 % en poids d'éthylhexylglycérol, par rapport au poids total de la composition cosmétique.

4. Composition cosmétique selon l'une des revendications 1 à 3, la teneur en hydroxyacétophénone étant de 0,2 à 1,0 % en poids, de préférence de 0,2 à 0,4 % en poids, par rapport au poids total de la composition cosmétique.

5. Composition cosmétique selon la revendication 1, dans laquelle seuls le 1,6-hexanediol et l'hydroxyacétophénone sont présents comme agents conservateurs, et dans laquelle la teneur en hydroxyacétophénone est de préférence de 0,4 à 0,8 % en poids, par rapport au poids total de la composition cosmétique.

6. Composition cosmétique selon l'une des revendications précédentes, dans laquelle la teneur en 1,6-hexanediol (b) est de 5 à 7 % en poids, de préférence de 5,5 à 6,5 % en poids, de manière davantage préférée de 6 % en poids, par rapport au poids total de la composition cosmétique.

7. Composition cosmétique selon l'une des revendications précédentes, la composition cosmétique étant un agent cosmétique destiné à réduire les signes du vieillissement de la peau.

8. Composition cosmétique selon l'une des revendications précédentes, la composition cosmétique contenant 0,01 % en poids ou moins d'autres agents conservateurs, de préférence aucun autre agent conservateur.

9. Utilisation de 1,6-hexanediol en combinaison avec l'hydroxyacétophénone comme système d'agents conservateurs pour des compositions cosmétiques, la concentration finale en 1,6-hexanediol dans la composition cosmétique étant de 3 à 12 % en poids, de préférence de 5 à 7 % en poids, de manière davantage préférée de 6 % en poids, et la concentration finale d'hydroxyacétophénone étant de 0,1 à 1,5 % en poids, de préférence de 0,2 à 0,8 % en poids, et le système d'agents conservateurs contenant éventuellement, en plus de l'hydroxyacétophénone et du 1,6-hexanediol, aussi du 2-phénoxyéthanol, de l'éthylhexylglycérol ou une combinaison de ceux-ci.

10. Utilisation de 1,6-hexanediol et d'hydroxyacétophénone, éventuellement en combinaison avec de l'éthylhexylglycérol ou du 2-phénoxyéthanol, pour tuer ou inhiber et/ou empêcher la croissance de bactéries et de champignons dans les agents cosmétiques, la concentration finale en 1,6-hexanediol dans la composition cosmétique étant de 3 à 12 % en poids, de préférence de 5 à 7 % en poids, de manière davantage préférée de 6 % en poids, et la concentration finale en hydroxyacétophénone étant de 0,1 à 1,5 % en poids, de préférence de 0,2 à 0,8 % en poids.
